(19) Europäisches Patentamt
European Patent Office
Office européen des brevets

(11) Veröffentlichungsnummer : **0 165 534**
**B1**

(12) # EUROPÄISCHE PATENTSCHRIFT

(45) Veröffentlichungstag der Patentschrift : **15.03.89**

(51) Int. Cl.$^4$ : **C 07 C163/00**, A 61 K 31/165

(21) Anmeldenummer : **85107095.3**

(22) Anmeldetag : **08.06.85**

(54) **S-(Carbamoyl-phenylselenyl)-Derivate des Glutathions und von Aminomercaptocarbonsäuren, Verfahren zu ihrer Herstellung und diese enthaltende pharmazeutische Präparate.**

(30) Priorität : **22.06.84 DE 3422962**
**29.11.84 DE 3443468**

(43) Veröffentlichungstag der Anmeldung : **27.12.85 Patentblatt 85/52**

(45) Bekanntmachung des Hinweises auf die Patenterteilung : **15.03.89 Patentblatt 89/11**

(84) Benannte Vertragsstaaten : **AT BE CH DE FR GB IT LI LU NL SE**

(56) Entgegenhaltungen : **DE--A-- 3 027 075**

(73) Patentinhaber : **A. Nattermann & Cie. GmbH**
**Nattermannallee 1**
**D-5000 Köln 30 (DE)**

(72) Erfinder : **Dereu, Norbert, Dr.**
**An der Holzhecke 11**
**D-5020 Frechen-Bachem (DE)**
Erfinder : **Welter, André, Dr.**
**Reiherweg 11a**
**D-5024 Pulheim (DE)**
Erfinder : **Wendel, Albrecht, Prof. Dr.**
**Im Buckenloh 19**
**D-7400 Tübingen 1 (DE)**
Erfinder : **Leyck, Sigurd, Dr.**
**Im Buckenloh 19**
**D-7400 Tübingen 1 (DE)**
Erfinder : **Parnham, Michael, Dr.**
**Aurikelweg 92**
**D-5024 Pulheim 1 (DE)**
Erfinder : **Graf, Erich, Dr.**
**An Langen Hau 25**
**D-5014 Kerpen (DE)**
Erfinder : **Sies, Helmut, Prof. Dr.**
**Wehler Dorfstrasse 32a**
**D-4040 Neuss 22 (DE)**
Erfinder : **Betzing, Hans, Dr.**
**Heidestock 7**
**D-5014 Kerpen-Horrem (DE)**
Erfinder : **Fischer, Hartmut, Dr.**
**Arnulfstrasse 3-5**
**D-5000 Köln 41 (DE)**

(74) Vertreter : **Redies, Bernd, Dr. rer. nat. et al**
**COHAUSZ & FLORACK Patentanwaltsbüro Schumannstrasse 97 Postfach 14 01 47**
**D-4000 Düsseldorf 1 (DE)**

**EP 0 165 534 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die vorliegende Erfindung betrifft neue S-(Carbamoyl-phenylselenyl)-Derivate des Glutathions und von α-Aminomercaptocarbonsäuren, die sich durch wertvolle pharmakologische Eigenschaften auszeichnen, sowie Verfahren zu ihrer Herstellung und ihre Verwendung als Wirkstoffe in Arzneimitteln. Sie können besonders zur Behandlung bei Krankheiten Verwendung finden, die durch eine Zellschädigung aufgrund vermehrter Bildung von aktiven Sauerstoffmetaboliten hervorgerufen werden, wie z. B. Leberschäden, Herzinfarkt, Entzündungen, Psoriasis, Strahlenschäden.

Die erfindungsgemäßen Verbindungen entsprechen der allgemeinen Formel I,

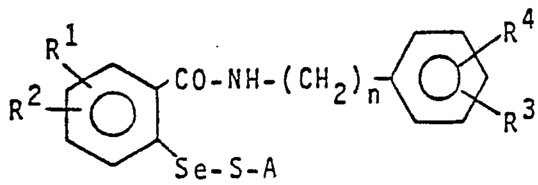

(I)

worin

$R^1$, $R^2$, $R^3$, $R^4$ gleich oder verschieden sind und unabhängig voneinander Wasserstoff, Halogen, $C_{1-4}$-Alkyl, $C_{1-4}$-Alkoxy, Hydroxy, Trifluormethyl, Nitro, Cyan, Carboxy, $C_{1-4}$-Alkoxycarbonyl bedeuten und

n Null oder eine ganze Zahl von 1-4 ist, während

A für den Glutathionrest oder für einen α-Aminosaäurerest steht, wobei die Carboxylgruppe mit einem $C_{1-3}$-Alkohol verestert und die Aminogruppe acyliert sein kann.

Halogen bedeutet Fluor, Chlor, Brom. Als Alkylreste mit 1-4 Kohlenstoffatomen seien Methyl, Ethyl, Propyl, Isopropyl, Butyl, Isobutyl, sec-Butyl, tert-Butyl genannt, als Alkoxyreste mit 1-4 Kohlenstoffatomen kommen Methoxy, Ethoxy, Propoxy, Butoxy in Betracht.

Bevorzugt sind dabei Verbindungen, in denen $R^1$, $R^2$, $R^3$, $R^4$ gleich oder verschieden sind und unabhängig voneinander Wasserstoff, Fluor, Chlor, Methyl, Methoxy, Hydroxy, Trifluormethyl oder Nitro bedeuten. Besonders bevorzugt sind dabei Verbindungen, in denen $R^1$, $R^2$ gleich oder verschieden sind und unabhängig voneinander Wasserstoff, Fluor, Chlor, Methyl, Methoxy, Hydroxy, Trifluormethyl oder Nitro bedeuten, während $R^3$, $R^4$ für Wasserstoff oder Methoxy stehen und A einen L-Glutathion- oder L-Methioninrest bedeutet.

Die Verbindungen der Formel I besitzen wegen der α-ständigen Aminogruppe im jeweiligen Aminosäurentest ein Chiralitätszentrum und können je nach Art der verwendeten Ausgangssubstanzen als Racemat oder in Form der D- oder L-Enantiomeren vorliegen. Falls eine Trennung der Racemate erwünscht ist, wird diese zweckmäßigerweise nach an sich bekannten Verfahren mit einer optisch aktiven Base über die Bildung diestereomerer Salze oder durch Chromatographie an optisch aktivem Säulenmaterial durchgeführt.

Erfindungsgemäße Verbindungen sind beispielsweise :

S-(2-Phenylcarbamoyl-phenylselenyl)-L-glutathion
S-[2-(2-Chlorphenylcarbamoyl)-phenylselenyl]-L-glutathion
S-[2-(3-Fluorphenylcarbamoyl)-phenylselenyl]-L-glutathion
S-[2-(4-Trifluormethylphenylcarbamoyl)-phenylselenyl]-L-glutathion
S-[2-(4-Hydroxyphenylcarbamoyl)-phenylselenyl]-L-glutathion
S-[2-(4-Methoxyphenylcarbamoyl)-phenylselenyl]-L-glutathion
S-[2-(3,4-Dichlorphenylcarbamoyl)-phenylselenyl]-L-glutathion
S-(2-Phenylcarbamoyl-5-methoxy-phenylselenyl)-L-glutathion
S-(2-Phenylcarbamoyl-5-chlor-phenylselenyl)-L-glutathion
S-(2-Phenylcarbamoyl-5-fluor-phenylselenyl)-L-glutathion
S-(2-Phenylcarbamoyl-6-methoxy-phenylselenyl)-L-glutathion
S-[2-(4-Nitrophenylcarbamoyl)-phenylselenyl]-L-glutathion
S-[2-(4-Chlorphenylcarbamoyl)-phenylselenyl]-L-glutathion
S-[2-(4-Fluorphenylcarbamoyl)-6-methoxy-phenylselenyl]-L-glutathion
S-[2-(4-Chlorphenylcarbamoyl)-6-methoxy-phenylselenyl]-L-glutathion
S-[2-(4-Methylphenylcarbamoyl)-6-methoxy-phenylselenyl]-L-glutathion
S-[2-(3-Chlorphenylcarbamoyl)-6-methoxy-phenylselenyl]-L-glutathion
S-[2-(3-Methoxyphenylcarbamoyl)-6-methoxy-phenylselenyl]-L-glutathion
S-[2-(3,4-Dichlorphenylcarbamoyl)-6-methoxy-phenylselenyl]-L-glutathion
S-[2-(3,4-Dichlorphenylcarbamoyl)-6-methoxy-phenylselenyl]-L-glutathion
S-[2-(3-Fluor-4-methyl-phenylcarbamoyl)-6-methoxy-phenylselenyl]-L-glutathion
S-[2-(3-Chlor-4-methoxy-phenylcarbamoyl)-6-methoxy-phenylselenyl]-L-glutathion
S-[2-(3,4-Dimethoxyphenylcarbamoyl)-6-methoxy-phenyl-selenyl]-L-glutathion

S-[2-(2-Methoxy-4-nitro-phenylcarbamoyl)-6-methoxy-phenylselenyl]-L-glutathin
S-(2-Phenylcarbamoyl-phenylselenyl)-L-cystein
S-(2-Phenylcarbamoyl-6-methoxy-phenylselenyl)-L-cystein
S-[2-(4-Carboxyphenylcarbamoyl)-phenylselenyl]-L-cystein
S-[2-(4-Ethoxycarbonylphenylcarbamoyl)-phenylselenyl]-L-cystein
S-[2-(4-Cyanphenylcarbamoyl)-phenylselenyl]-L-cystein
S-(2-Benzylcarbamoyl-phenylselenyl)-L-cystein
S-[2-(4-Phenylbutylcarbamoyl)-phenylselenyl]-L-cystein
S-(2-Phenylcarbamoyl-phenylselenyl)-L-cysteinmethylester
S-(2-Phenylcarbamoyl-phenylselenyl)-L-cysteinethylester
S-(2-Phenylcarbamoyl-phenylselenyl)-L-cysteinpropylester
S-[2-(4-Trifluormethylphenylcarbamoyl)-phenylselenyl]-L-cysteinmethylester
S-(2-Phenylcarbamoyl-phenylselenyl)-N-acetyl-L-cystein
S-[2-(2-Chlorphenylcarbamoyl)-phenylselenyl]-N-acetyl-L-cystein
S-[2-(4-Methoxyphenylcarbamoyl)-phenylselenyl]-N-acetyl-L-cystein
S-[3-(4-Fluorphenylcarbamoyl)-phenylselenyl]-N-acetyl-L-cystein
S-[2-(4-Fluorphenylcarbamoyl)-6-methoxy-phenylselenyl]-N-acetyl-L-cysteinmethylester
S-(2-Phenylcarbamoyl-phenylselenyl)-DL-homocystein
S-(2-Phenylcarbamoyl-6-methoxy-phenylselenyl)-DL-homocystein
S-[2-(4-Carboxyphenylcarbamoyl)-phenylselenyl]-DL-homocystein
S-[2-(4-Methoxycarbonylphenylcarbamoyl)-phenylselenyl]-DL-homocystein
S-[2-(4-Cyanphenylcarbamoyl)-phenylselenyl]-DL-homocystein
S-(2-Benzylcarbamoyl-phenylselenyl)-DL-homocystein
S-[2-(4-Phenylbutylcarbamoyl)-phenylselenyl]-DL-homocystein
S-(2-Phenylcarbamoyl-phenylselenyl)-DL-homocysteinethylester
S-(2-Phenylcarbamoyl-phenylselenyl)-N-acetyl-DL-homocystein
S-(2-Phenylcarbamoyl-6-methoxy-phenylselenyl)-N-acetyl-DL-homocystein
S-(2-Phenylcarbamoyl-phenylselenyl)-N-acetyl-DL-homocysteinmethylester
S-(2-Phenylcarbamoyl-phenylselenyl)-D-penicillamin
S-(2-Phenylcarbamoyl-6-methoxy-phenylselenyl)-D-penicillamin
S-[2-(4-Carboxyphenylcarbamoyl)-phenylselenyl]-D-penicillamin
S-[2-(4-Ethoxycarbonylphenylcarbamoyl)-phenylselenyl]-D-penicillamin
S-[2-(4-Cyanphenylcarbamoyl)-phenylselenyl]-D-penicillamin
S-(2-Benzylcarbamoyl-phenylselenyl)-D-penicillamin
S-[2-(4-Phenylbutylcarbamoyl)-phenylselenyl]-D-penicillamin
S-(2-Phenylcarbamoyl-phenylselenyl)-D-penicillaminmethylester
S-(2-Phenylcarbamoyl-6-methoxy-phenylselenyl)-D-penicillaminmethylester
S-(2-Phenylcarbamoyl-phenylselenyl)-N-acetyl-D-penicillamin
S-(2-Phenylcarbamoyl-6-methoxy-phenylselenyl)-N-acetyl-D-penicillamin
S-(2-Phenylcarbamoyl-phenylselenyl)-N-acetyl-D-penicillaminmethylester

Die erfindungsgemäßen Substanzen weisen glutathionperoxidaseartige Eigenschaften auf und sind damit in der Lage, dieses Enzym zu ersetzen und auf diese Weise im Zusammenwirkung mit einem Mercaptan die schädigende Wirkung aktiver Sauerstoffmetaboliten zu verhindern.

Die selenabhängige Glutathion(GSH)-Peroxidase (Px) katalysiert die Reduktion von $H_2O_2$ und organischen Hydroperoxiden.

$$2\ GSH + H_2O_2 \xrightarrow{GSH\text{-}Px} GSSG$$

$$2\ GSH + ROOH \xrightarrow{GSH\text{-}Px} GSSG + ROH + H_2O.$$

Das selenhaltige Enzym schützt die Zellen gegen Peroxidation und spielt eine wichtige Rolle in der Modulierung des Arachidonsäure-Stoffwechsels (C. C. Reddy, E. J. Massaro, Fundam. and Appl. Toxicology (3), 9-10 (1983), S. 431-436 und L. Flohé in Free Radicals in Biology, Vol. V, Edited by W. A. Pryor 1982 Academic Press, S. 223-254).

Die Glutathion-Peroxidase spielt bei allen Erkrankungen eine Rolle, bei denen es zu einer Zellschädigung des betreffenden Gewebes und schließlich zur Nekrose aufgrund einer vermehrten Bildung von aktiven Sauerstoffmetaboliten in Form von Peroxiden (z. B. Lipidperoxide und Wasserstoffperoxid) kommt. Dieser sogenannte « oxidative Streß » wird z. B. beobachtet bei Lebererkrankungen — induziert durch entzündliche oder autoimmunologische Reaktionen, durch Alkohol oder durch Medikamente — aber auch bei anderen Erkrankungen, wie z. B. Herzinfarkt. Es ist bekannt, daß nach einem Herzinfarkt in das geschädigte Gebiet Leukozyten einwandern und der Zelluntergang von einer vermehrten Freisetzung der genannten aktiven Sauerstoffmetaboliten begleitet ist. Dies führt schließlich zu einem fortschreitenden Gewebeabbau.

In solchen Fällen ist das hierfür wichtige und natürlicherweise vorhandene Schutzsystem, bestehend

aus verschiedenen, Peroxide und aktiven Sauerstoff abbauenden Enzymen, überfordert. Hierzu gehören Superoxiddismutase, Katalase, und besonders das Glutathion-Redox-System mit der dazugehörenden Enzymkomponente Glutathion-Peroxidase. Gerade diesem letzteren Prinzip kommt eine große Bedeutung zu, da es sowohl organische Peroxide als auch Wasserstoffperoxid entgiften kann. Es ist belegt, daß dieses System für die intakte Leberfunktion eine große Rolle spielt (Wendel et al.: Biochemical Pharmacology, Vol. 31, S. 3601 (1982)) und z. B. das Ausmaß eines experimentellen Leberschadens gerade von diesem System abhängt, d. h. von dem Gehalt der Leber an Glutathion einerseits und von der Aktivität des Enzyms Glutathion-Peroxidase andererseits. Im Verlauf einer allgemeinen Entzündung wird dieser Leberschutzmechanismus stark reduziert (Bragt et al., Agents and Actions, Supp. 17, S. 214 (1980)), wodurch die Leber einen stark erhöhten « oxidativen Streß » erleidet.

Eine sehr wichtige Rolle spielen die reaktiven Sauerstoffmetaboliten als Mediatoren von Entzündungen. Sie scheinen sowohl bei Leucotaxis, Gefäßdurchlässigkeit, Bindegewebsschäden und Immunkomplex/Komplement-induzierten Effekten mitzuwirken als auch bei Schäden, wie sie durch Wiedereinströmen in ischämischen Bereichen entstehen (L. Flohé et al., in The Pharmacology of Inflammation, ed. I. L. Bonta et al., Handbook of Inflammation, Vol. 5, Elsevier, Amsterdam, S. 255-270).

Auch die Schäden nach ionisierender Bestrahlung sind auf die Bildung von Radikalen und aktiven Sauerstoffmetaboliten zurückzuführen. Ein Weg für die chemische Zytoprotektion besteht somit in der Stärkung des Glutathion/Glutathionperoxidase-Systems.

Die Messung der glutathionperoxidaseartigen Aktivität erfolgte nach der Methode von A. Wendel (A. Wendel, Methods in Enzymology Vol. 77, 325-333 (1981)). Gemessen wird in diesem Versuch die Umsetzung des Co-Substrats Nicotinamid-adenin-dinucleotid-phosphat. Als Reduktionsmittel diente in diesem Falle nicht Glutathion, sondern die mercaptanhaltige Aminosäure, die für die Synthese der jeweiligen Verbindung verwendet wurde. Es wurde nun überraschenderweise gefunden, daß die erfindungsgemäßen Verbindungen der Formel I eine glutathionperoxidaseartige Wirkung besitzen. Die Reaktion der Benzisoselenazolone mit mercaptanhaltigen Aminosäuren erfolgt am Beispiel des 2-Phenyl-1,2-benzisoselenazol-3(2H)-on nach folgender Gleichung:

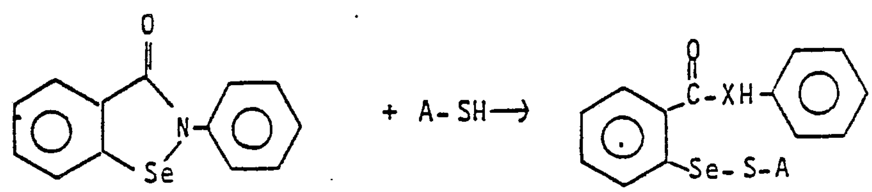

A = Aminosäurerest

Glutathionperoxidaseartige Wirkung

Bei in vitro-Untersuchungen wurde die Katalyse des Peroxidaseabbaus geprüft. Dabei wurde festgestellt, daß die erfindungsgemäßen Verbindungen die Glutathion-Peroxidase ersetzen können.

$$\begin{array}{lcl} ROOH & \xrightarrow[\text{Verbindungen}]{\text{Erfindungsgemäße}} & ROH \\ H_2O_2 & & H_2O \\ RSH & & RSSR \end{array}$$

Die Reaktionsgeschwindigkeiten wurden nach der Methode von A. Wendel ermittelt (A. Wendel, Methods in Enzymology Vol. 77, S. 325-333 (1981)). Als Referenzsubstanz wurde S-(2-Phenylcarbamoyl-phenylselenyl)-glutathion benutzt. In Gegenwart einer 1 mmolaren Konzentration an Glutathion wird mit tert-Butylhydroperoxid eine Reaktionsgeschwindigkeit von $1{,}17 \times 10^6$ Einheiten pro Mol beobachtet. Diese Aktivität wird zum besseren Vergleich als 100 % eingesetzt.

| | Katalytische Aktivität (%) |
|---|---|
| S-(2-Phenylcarbamoyl-phenylselenyl)-L-glutathion | 100 |
| S-[2-(2-Chlorphenylcarbamoyl)-phenylselenyl]-L-glutathion | 39 |
| S-[2-(2-Methoxyphenylcarbamoyl)-phenylselenyl]-L-glutathion | 37 |
| S-[2-(3-Fluorphenylcarbamoyl)-phenylselenyl]-L-glutathion | 113 |
| S-[2-(4-Trifluormethylphenylcarbamoyl)-phenylselenyl]-L-glutathion | 49 |
| S-[2-(4-Methoxyphenylcarbamoyl)-phenylselenyl]-L-glutathion | 124 |
| S-[2-(3,4-Dichlorphenylcarbamoyl)-phenylselenyl]-L-glutathion | 21 |
| S-(2-Phenylcarbamoyl-5-methoxy-phenylselenyl)-L-glutathion | 63 |

| | |
|---|---|
| S-(2-Phenylcarbamoyl-5-chlor-phenylselenyl)-L-glutathion | 99 |
| S-(2-Phenylcarbamoyl-5-fluor-phenylselenyl)-L-glutathion | 92 |
| S-(2-Phenylcarbamoyl-6-methoxy-phenylselenyl)-L-glutathion | 225 |
| S-[2-(4-Nitrophenylcarbamoyl)-phenylselenyl]-L-glutathion | 121 |
| S-[2-(4-Chlorphenylcarbamoyl)-phenylselenyl]-L-glutathion | 116 |
| S-(2-Phenylcarbamoyl-phenylselenyl)-L-cystein | 210 |
| S-(2-Phenylcarbamoyl-phenylselenyl)-N-acetyl-L-cystein | 40 |
| S-(2-Phenylcarbamoyl-phenylselenyl)-D-penicillamin | 25 |
| S-(2-Benzylcarbamoyl-phenylselenyl)-L-cystein | 56 |
| S-[2-(4-Fluorphenylcarbamoyl)-phenylselenyl]-N-acetyl-L-cystein | 61 |
| S-(2-Phenylcarbamoyl-6-methoxy-phenylselenyl)-L-cystein | 440 |

Die Herstellung der erfindungsgemäßen Verbindungen erfolgt durch Umsetzung von 1,2-Benzisoselenazolonen der Formel II, die nach der Vorschrift von DE-OS 30 27 073 = USP 4.352.799 bzw. DE-OS 30 27 075 = USP 4.418.069 erhalten werden, mit Glutathion bzw. mit α-Aminomercaptocarbonsäuren oder deren Derivaten.

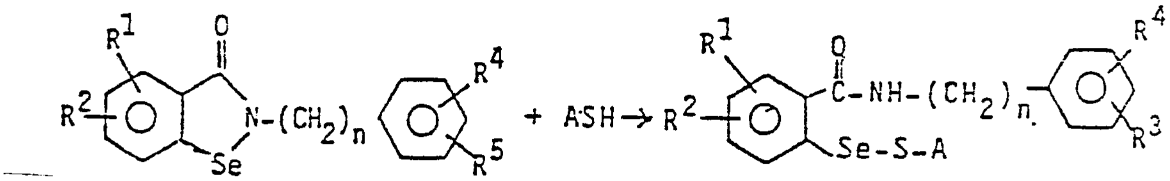

(II) (I)

Die Umsetzung erfolgte unter Rühren bei Raumtemperatur in Wasser bzw. mit Wasser mischbaren Lösungsmitteln. Die für die Umsetzung verwendeten -Amino-mercaptocarbonsäuren bzw. Glutathion sind bekannte Substanzen.

Die vorliegende Erfindung betrifft ebenfalls pharmazeutische Präparate, welche Verbindungen der Formel I enthalten. Bei den erfindungsgemäßen pharmazeutischen Präparaten handelt es sich um solche zur enteralen wie oralen oder rektalen sowie parenteralen Verabreichung, welche die pharmazeutischen Wirkstoffe allein oder zusammen mit einem üblichen, pharmazeutisch anwendbaren Trägermaterial enthalten. Vorteilhafterweise liegt die pharmazeutische Zubereitung des Wirkstoffes in Form von Einzeldosen vor, die auf die gewünschte Verabreichung abgestimmt sind, z. B. Tabletten, Dragées, Kapseln, Suppositorien, Granulate, Lösungen, Emulsionen oder Suspensionen. Die Dosierung der Substanzen liegt überlicherweise zwischen 10 und 1 000 mg pro Tag, vorzugsweise zwischen 30 und 300 mg pro Tag, und kann in einer Dosis oder mehreren Teildosen, vorzugsweise in zwei bis drei Teildosen pro Tag, verabreicht werden.

Die Herstellung der erfindungsgemäßen Verbindungen wird durch die folgenden Beispiele näher erläutert. Die angegebenen Schmelzpunkte wurden mit einem Büchi 510-Schmelzpunktbestimmungsapparat gemessen und sind mit °C angegeben und nicht korrigiert.

## Beispiel 1

S-(2-Phenylcarbamoyl-phenylselenyl)-L-glutathion. 1,1 g L-Glutathion (4 mmol) werden in 20 ml Wasser gelöst. Dazu wird eine Lösung von 1 g 2-Phenyl-1,2-benzisoselenazol-3(2H)-on (3,65 mmol) in 10 ml Dimethylformamid unter Rühren langsam zugetropft. Die zunächst trübe Lösung wird nach vollständiger Zugabe klar und gelb. Nach einigen Minuten fällt ein Niederschlag aus. Die Suspension wird über Nacht weitergerührt. Der Niederschlag wird dann abgesaugt, mit 50 ml Wasser, dann mit 20 ml Ethanol gewaschen und getrocknet.

Ausbeute : 1,95 g (92,8 % d. Th.), Fp. 245-247 (Zers.)

| Analyse | berechnet | gefunden |
|---|---|---|
| C | 47,5 % | 47,49 % |
| H | 4,5 % | 4,53 % |
| N | 9,6 % | 9,48 % |
| S | 5,5 % | 6,07 % |
| Se | 13,6 % | 14,40 % |

Im $^{77}Se$ NMR-Spektrum kann man für 2-Phenyl-1,2-benzisoselenazol-3(2H)-on ein Signal bei 959 ppm (Referenz $CH_3$-Se-$CH_3$) beobachten. Dieses Signal ist im Umsetzungsprodukt nicht mehr

vorhanden. Dagegen wird ein neues Signal bei 394 ppm sichtbar. Diese chemische Verschiebung ist charakteristisch für Selenenylsulfide.

Beispiel 2

S-[2-(4-Methoxyphenylcarbamoyl)-phenylselenyl]-L-glutathion

Analog Beispiel 1 werden 1,1 g L-Glutathion (4,0 mmol) in 20 ml Wasser mit einer Lösung von 1,1 g 2-(4-Methoxyphenyl)-1,2-benzisoselenazol-3(2H)-on (3,62 mmol) in 10 ml Dimethylformamid behandelt. Der Niederschlag wird über Nacht gerührt und abgesaugt, mit 50 ml Wasser, dann mit 20 ml Ethanol gewaschen und getrocknet.
Ausbeute : 1,5 g (67,7 % d. Th.), Fp. 238-243 °C.

Analyse : Se
berechnet : 12,9 %
gefunden : 13,4 %

Beispiel 3

S-[2-(4-Chlorphenylcarbamoyl)-phenylselenyl]-L-glutathion

Analog Beispiel 1 werden 1,1 g L-Glutathion (4,0 mmol) in 20 ml Wasser mit einer Lösung von 1,2 g 2-(4-Chlorphenyl)-1,2-benzisoselenazol-3(2H)-on in 10 ml Dimethylformamid behandelt. Nach der üblichen Aufarbeitung erhält man 1,1 g (45,9 % d. Th.), Fp. 279-283 °C

Analyse : Se
berechnet : 12,8 %
gefunden : 12,9 %

Beispiel 4

S-[2-(3-Methoxyphenylcarbamoyl)-phenylselenyl]-L-glutathion

Analog Beispiel 1 werden aus 1 g 2-(3-Methoxyphenyl)-1,2-benzisoselenazol-3(2H)-on (3,29 mmol) erhalten.
Ausbeute : 1,4 g (69,5 % d. Th.), Fp. 222-225 °C

Analyse : Se
berechnet : 12,9 %
gefunden : 13,8 %

Beispiel 5

S-[2-(4-Cyanophenylcarbamoyl)-phenylselenyl]-L-glutathion

Analog Beispiel 1 werden aus 1,1 g 2-(4-Cyanophenyl)-1,2-benzisoselenazol-3(2H)-on (3,67 mmol) erhalten.
Ausbeute : 1,25 g (56,1 % d. Th.), Fp. 227-235 °C (Zers.)

Analyse : Se
berechnet : 13,0 %
gefunden : 13,9 %

Beispiel 6

S-(2-Phenylcarbamoyl-5-fluor-phenylselenyl)-L-glutathion

Analog Beispiel 1 werden aus 1,1 g 6-Fluor-2-Phenyl-1,2-benzisoselenazol-3(2H)-on (3,77 mmol) erhalten.
Ausbeute : 1,75 g (77,4 % d. Th.), Fp. 250-270 °C (Zers.)

Analyse : Se
berechnet : 13,2 %
gefunden : 13,7 %

Beispiel 7

S-[2-(3,4-Dichlorphenylcarbamoyl)-phenylselenyl]-L-glutathion

Analog Beispiel 1 werden aus 1,0 g 2-(3,4-Dichlorphenyl)-1,2-benzisoselenazol-3(2H)-on (2,91 mmol) erhalten.
Ausbeute : 1,65 g (87 % d. Th.), Fp. 265-271 °C

Analyse : Se
berechnet : 12,1 %
gefunden :12,6 %

Beispiel 8

S-[2-(4-Chlor-3-methoxy-phenylcarbamoyl)-phenylselenyl]-L-glutathion

Analog Beispiel 1 werden aus 1,2 g 2-(4-Chlor-3-methoxy-phenyl)-1,2-benzisoselenazol-3(2H)-on (3,54 mmol) erhalten.
Ausbeute : 1,7 g (74,3 % d. Th.), Fp. 240-248 °C (Zers.)

Analyse : Se
berechnet : 12,2 %
gefunden : 12,5 %

Beispiel 9

S-(2-Phenylcarbamoyl-6-methoxy-phenylselenyl)-L-glutathion

Analog Beispiel 1 werden aus 1,4 g 7-Methoxy-2-phenyl-1,2-benzisoselenazol-3(2H)-on (4,61 mmol) erhalten.
Ausbeute : 1,85 g (65,6 % d. Th.), Fp. 235-241 °C (Zers.)

Analyse : Se
berechnet : 12,9 %
gefunden : 12,8 %

Beispiel 10

S-[2-(3-Fluorphenylcarbamoyl)-phenylselenyl]-L-glutathion

Analog Beispiel 1 werden aus 1,65 g 2-(3-Fluorphenyl)-1,2-benzisoselenazol-3(2H)-on (5,65 mmol) erhalten.
Ausbeute : 2,1 g (62 % d. Th.), Fp. 268-274 °C (Zers.)

Analyse : Se
berechnet : 13,2 %
gefunden : 12,9 %

Beispiel 11

S-(2-Phenylcarbamoyl-phenylselenyl)-L-cystein

2,74 g (10 mmol) 2-Phenyl-1,2-benzisoselenazol-3(2H)-on und 1,21 g (10 mmol) L-Cystein werden in 25 ml Trifluoressigsäure gelöst. Diese Lösung wird 18 Stunden bei Raumtemperatur gerührt. Zu dieser Lösung werden dann 150 ml eines Eis-Wassergemischs hinzugegeben. Der ausgefallene Niederschlag wird nach einigem Rühren fest. Er wird abgesaugt und schließlich in Methanol aufgenommen. Nach Eindampfen des Lösungsmittels verbleibt ein Rückstand, der mit Ether nachgewaschen wird.
Ausbeute : 3,85 g (97,5 % d. Th.), Fp. 250 °C (Zers.)

Beispiel 12

S-(2-Phenylcarbamoyl-phenylselenyl)-D-penicillamin

5 g (18,2 mmol) 2-Phenyl-1,2-benzisoselenazol-3(2H)-on werden in 250 ml Methanol und 30 ml Dimethylformamid gelöst. Dazu wird eine Lösung von 2,71 g (18,2 mmol) D-Penicillamin in 100 ml Wasser

unter Rühren zugetropft. Dieser klaren Lösung wird soviel Wasser zugegeben, bis eine leichte Trübung zu beobachten ist. Nach 18 Stunden Rühren wird der Niederschlag abfiltriert und aus Ethanol/Wasser 3 : 2 umkristallisiert.

Ausbeute : 6,1 g (79 % d. Th.), Fp. 265-266 °C

$[\alpha]^{20}$ $Na_{589}$ : + 67,5
$Hg_{578}$ : + 69,6

Beispiel 13

S-(2-Phenylcarbamoyl-phenylselenyl)-D,L-penicillamin

Analog Beispiel 12
Ausbeute : 5,95 g (77,2 % d. Th.), Fp. 255-256 °C

Beispiel 14

S-(2-Phenylcarbamoyl-phenylselenyl)-N-acetyl-L-cystein

Analog Beispiel 12 aus :
1 g (3,65 mmol) 2-Phenyl-1,2-benzisoselenazol-3(2H)-on in 50 ml Methanol und 6 ml Dimethylformamid und 0,6 g (3,67 mmol) N-Acetyl-L-cystein in 20 ml Wasser.
Ausbeute : 0,95 g (60 % d. Th.), Fp. 167-168 °C

Beispiel 15

S-(2-Phenylcarbamoyl-phenylselenyl)-L-cysteinethylester

Analog Beispiel 11 aus :
2,2 g (8 mmol) 2-Phenyl-1,2-benzisoselenazol-3(2H)-on und 1,2 g (8 mmol) L-Cysteinethylester in 20 ml Trifluoressigsäure.
Ausbeute : 2,2 g (65 % d. Th.), Fp. 230 °C (Zers.)

Beispiel 16

S-[2-(4-Fluorphenylcarbamoyl)-phenylselenyl]-N-acetyl-L-cystein

Analog Beispiel 12 aus :
1 g (3,42 mmol) 2-(4-Fluorphenyl)-1,2-benzisoselenzol-3(2H)-on in 50 ml Methanol und 6 ml Dimethylformamid und 0,6 g (3,67 mmol) N-Acetyl-L-cystein in 20 ml Wasser.
Ausbeute : 1,5 g (96 % d. Th.), Fp. 110 °C

Beispiel 17

S-[2-(4-Nitrophenylcarbamoyl)-phenylselenyl]-D-penicillamin

Analog Beispiel 12 aus :
1 g (3,13 mmol) 2-(4-Nitrophenyl)-1,2-benzisoselenazol-3(2H)-on in 50 ml Methanol und 6 ml Dimethylformamid und 0,5 g (3,35 mmol) D-Penicillamin in 20 ml Wasser.
Ausbeute : 0,63 g (43 % d. Th.), Fp. 158 °C (Zers.)

Beispiel 18

S-(2-Phenylcarbamoyl-6-methoxy-phenylselenyl)-N-acetyl-L-cystein

Analog Beispiel 12 aus :
1 g (3,29 mmol) 2-Phenyl-7-methoxy-1,2-benzisoselenazol-3(2H)-on in 50 ml Methanol und 6 ml Dimethylformamid und 0,55 g (3,37 mmol) N-Acetyl-L-cystein in 20 ml Wasser.
Ausbeute : 1,45 g (94,4 % d. Th.), Fp. 181 °C

Beispiel 19

S-(2-Phenylcarbamoyl-phenylselenyl)-DL-homocystein

Analog Beispiel 11 aus :

2,74 g (10 mmol) 2-Phenyl-1,2-benzisoselenazol-3(2H)-on und 1,35 g (10 mmol) DL-Homocystein. Ausbeute : 3,70 g (90,5 % d. Th.), Fp. 270 °C (Zers.)

Beispiel 20

S-(2-Benzylcarbamoyl-phenylselenyl)-D-penicillamin

Analog Beispiel 12 aus :
2,88 g (10 mmol) 2-Benzyl-1,2-benzisoselenazol-3(2H)-on und 1,5 g (10 mmol) D-Penicillamin. Ausbeute : 4,3 g (98,4 % d. Th.), Fp. 182 °C

Beispiel 21

S-(2-Benzylcarbamoyl-phenylselenyl)-L-cystein

Analog Beispiel 11 aus :
2,88 g (10 mmol) 2-Benzyl-1,2-benzisoselenazol-3(2H)-on und 1,21 g (10 mmol) L-Cystein. Ausbeute : 4,0 g (97,8 % d. Th.), Fp. 192-194 °C.

Beispiel 22

S-(2-Benzylcarbamoyl-phenylselenyl)-L-cysteinethylester

Analog Beispiel 11 aus :
2,88 g (10 mmol) 2-Benzyl-1,2-benzisoselenazol-3(2H)-on und 1,5 g (10 mmol) L-Cysteinethylester. Ausbeute : 3,9 g (89 % d. Th.), Fp. 220 °C (Zers.)

**Patentansprüche** (für die Vertragsstaaten : BE, CH, DE, FR, GB, IT, LI, LU, NL, SE)

1. S-(Carbamoyl-phenylselenyl)-Derivate des Glutathions und von α-Amino-mercaptocarbonsäuren der allgemeinen Formel I

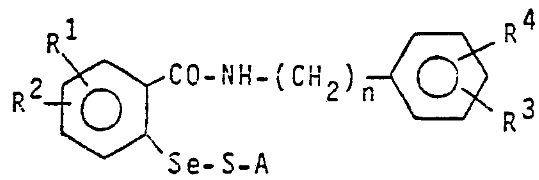

(I)

worin
$R^1$, $R^2$, $R^3$, $R^4$ gleich oder verschieden sind und unabhängig voneinander Wasserstoff, Halogen, $C_{1-4}$-Alkyl, $C_{1-4}$-Alkoxy, Trifluormethyl, Nitro, Cyan, Hydroxy, Carboxy, $C_{1-4}$-Alkoxycarbonyl bedeuten und
n Null oder eine ganze Zahl von 1 bis 4 ist, während
A für den Glutathionrest oder für einen α-Aminosäurerest steht, wobei die Carboxylgruppe mit einem $C_{1-3}$-Alkohol verestert und die Aminogruppe acyliert sein kann.

2. S-(Carbamoyl-phenylselenyl)-Derivate des L-Glutathions gemäß Formel I, Anspruch 1, worin
$R^1$, $R^2$ gleich oder verschieden sind und unabhängig voneinander Wasserstoff, Fluor, Chlor, Methyl, Methoxy, Hydroxy, Trifluormethyl, Nitro und
$R^3$, $R^4$ gleich oder verschieden sind und unabhängig voneinander Wasserstoff, Fluor, Chlor, Methoxy, Hydroxy bedeuten und
n Null ist, während
A für den L-Glutathionrest steht.

3. S-(Carbamoyl-phenylselenyl)-Derivate des L-Cysteins gemäß Formel I, Anspruch 1, worin
$R^1$, $R^2$ gleich oder verschieden sind und unabhängig voneinander Wasserstoff, Fluor, Chlor, Methyl, Methoxy, Hydroxy, Trifluormethyl, Nitro und
$R^3$, $R^4$ gleich oder verschieden sind und unabhängig voneinander Wasserstoff, Fluor, Chlor, Methyl, Methoxy, Hydroxy bedeuten und
n Null oder eine ganze Zahl von 1 bis 4 ist, während
A für den L-Cysteinrest steht.

4. Verfahren zur Herstellung von Verbindungen der Formel I gemäß den Ansprüchen 1-3, dadurch gekennzeichnet, daß man in einem mit Wasser mischbaren Lösungsmittel in annähernd äquimolarer Menge gelöstes 1,2-Benzisoselenazolon der Formel II

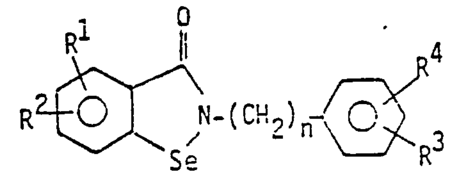

(II)

in der $R^1$, $R^2$, $R^3$, $R^4$ und n die in Formel I angegebenen Bedeutungen haben, mit der wäßrigen Lösung der jeweiligen mercaptogruppenhaltigen Verbindung der Formel III

A—SH (III)

unter Rühren bei Raumtemperatur umsetzt, wobei A die in Formel I angegebene Bedeutung hat.

5. Pharmazeutische Präparate, dadurch gekennzeichnet, daß sie eine Verbindung der Formel I gemäß den Ansprüchen 1-3 als Wirkstoff im Gemisch mit üblichen pharmazeutischen Hilfs- und Trägerstoffen enthalten.

**Patentansprüche** (für den Vertragsstaat AT)

1. Verfahren zur Herstellung von S-(Carbamoyl-phenylselenyl)-Derivaten des Glutathions und von α-Amino-mercaptocarbonsäuren der allgemeinen Formel I

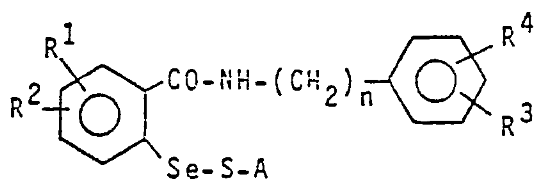

(I)

worin

$R^1$, $R^2$, $R^3$, $R^4$ gleich oder verschieden sind und unabhängig voneinander Wasserstoff, Halogen, $C_{1-4}$-Alkyl, $C_{1-4}$-Alkoxy, Trifluormethyl, Nitro, Cyan, Hydroxy, Carboxy, $C_{1-4}$-Alkoxycarbonyl bedeuten und

n Null oder eine ganze Zahl von 1 bis 4 ist, während

A für den Glutathionrest oder für einen α-Aminosäurerest steht, wobei die Carboxylgruppe mit einem $C_{1-3}$-Alkohol verestert und die Aminogruppe acyliert sein kann,

dadurch gekennzeichnet, daß man in einem mit Wasser mischbaren Lösungsmittel in annähernd äquimolarer Menge gelöstes 1,2-Benzisoselenazolon der Formel II

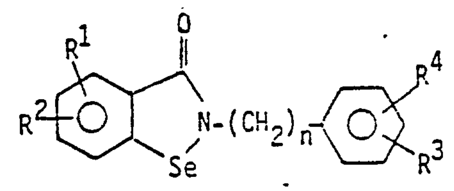

(II)

in der $R^1$, $R^2$, $R^3$, $R^4$ und n die in Formel I angegebenen Bedeutungen haben, mit der wäßrigen Lösung der jeweiligen mercaptogruppenhaltigen Verbindung der Formel III

A—SH (III)

unter Rühren bei Raumtemperatur umsetzt, wobei A die in Formel I angegebene Bedeutung hat.

2. Verfahren gemäß Anspruch 1, dadurch gekennzeichnet, daß von Verbindungen der Formeln II und III ausgegangen wird, worin

$R^1$, $R^2$ gleich oder verschieden sind und unabhängig voneinander Wasserstoff, Fluor, Chlor, Methyl, Methoxy, Hydroxy, Trifluormethyl, Nitro und

$R^3$, $R^4$ gleich oder verschieden sind und unabhängig voneinander Wasserstoff, Fluor, Chlor, Methoxy, Hydroxy bedeuten und

n Null ist, während

A für den L-Glutathionrest steht.

3. Verfahren gemäß Anspruch 1, dadurch gekennzeichnet, daß von Verbindungen der Formeln II und III ausgegangen wird, worin

$R^1$, $R^2$ gleich oder verschieden sind und unabhängig voneinander Wasserstoff, Fluor, Chlor, Methyl, Methoxy, Hydroxy, Trifluormethyl, Nitro und

$R^3$, $R^4$ gleich oder verschieden sind und unabhängig voneinander Wasserstoff, Fluor, Chlor, Methyl, Methoxy, Hydroxy bedeuten und

n Null oder eine ganze Zahl von 1 bis 4 ist, während
A für den L-Cysteinrest steht.

**Claims** (for the Contracting States : BE, CH, DE, FR, GB, IT, LI, LU, NL, SE)

1. S-(Carbamoyl-phenylselenyl) derivatives of glutathione and of α-aminomercaptocarboxylic acids represented by the general formula (I)

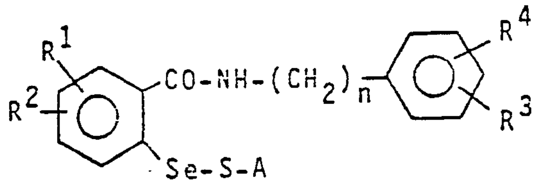

(I)

wherein

$R^1$, $R^2$, $R^3$, $R^4$, which can be identical or different, are independently hydrogen, halogen, $C_{1-4}$-alkyl, $C_{1-4}$-alkoxy, trifluoromethyl, nitro, cyano, hydroxy, carboxy, $C_{1-4}$-alkoxycarbonyl and

n is zero or an integer from 1 to 4, while

A represents the glutathione radical or an α-amino acid radical, wherein the carboxylic group can be esterified with a $C_{1-3}$-alcohol and wherein the amino group can be acylated.

2. S-(Carbamoyl-phenylselenyl) derivatives of L-glutathione according to formula (I) in claim 1, wherein

$R^1$, $R^2$ are identical or different and independently hydrogen, fluorine, chlorine, methyl, methoxy, hydroxy, trifluoromethyl, nitro and

$R^3$, $R^4$ are identical or different and independently hydrogen, fluorine, chlorine, methoxy, hydroxy and

n is zero, while

A represents the L-glutathione radical.

3. S-(Carbamoyl-phenylselenyl) derivatives of L-cysteine according to formula (I) of claim 1, wherein :

$R^1$, $R^2$ are identical or different and independently hydrogen, fluorine, chlorine, methyl, methoxy, hydroxy, trifluoromethyl, nitro and

$R^3$, $R^4$ are identical or different and independently hydrogen, fluorine, chlorine, methyl, methoxy, hydroxy and

n is zero or an integer from 1 to 4, while

A represents the L-cysteine radical.

4. A process for the preparation of compounds of formula (I) according to any of claims 1 to 3, characterized in that 1,2-benzoisoselenazolone of the formula (II)

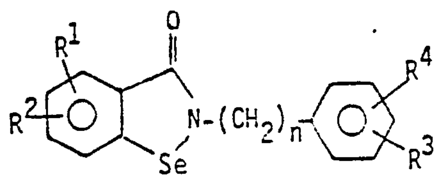

(II)

wherein $R^1$, $R^2$, $R^3$, $R^4$ and n have the meanings given in formula (I), dissolved in a solvent miscible with water in an about equimolar amount is reacted with the aqueous solution of the respective mercapto group containing compound of formula (III)

$$A—SH \quad (III)$$

at room temperature while stirring, wherein A has the meaning given in formula (I).

5. A pharmaceutical preparation, characterized in that it contains a compound of formula (I) according to any of the claims 1 to 3 as active component in mixture with common pharmaceutical excipients and carriers.

**Claims** (for the Contracting State AT)

1. Process for the production of S-(carbamoyl-phenylselenyl) derivatives of glutathione and of α-aminomercaptocarboxylic acids represented by the general formula I

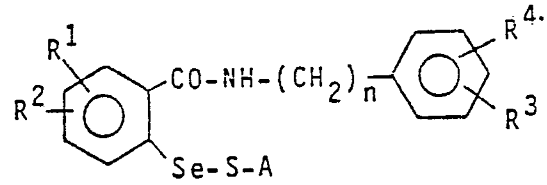

(I)

wherein

$R^1$, $R^2$, $R^3$, $R^4$, which can be identical or different, are independently hydrogen, halogen, $C_{1-4}$-alkyl, $C_{1-4}$-alkoxy, trifluoromethyl, nitro, cyano, hydroxy, carboxy, $C_{1-4}$-alkoxycarbonyl and

n is zero or an integer from 1 to 4, while

A represents the glutathione radical or an α-amino acid radical, wherein the carboxylic group can be esterified with a $C_{1-3}$-alcohol and wherein the amino group can be acylated,

characterized in that 1,2-benzoisoselenazolone of the formula II

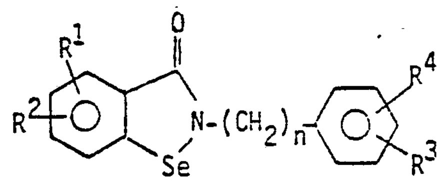

(II)

wherein $R^1$, $R^2$, $R^3$, $R^4$ and n have the meanings given in formula I, dissolved in a solvent miscible with water in an about equimolar amount is reacted with the aqueous solution of the respective mercapto group containing compound of formula III

$$A\text{—}SH \qquad (III)$$

at room temperature while stirring, wherein A has the meaning given in formula I.

2. Process according to claim 1, characterized in that it is started from compounds of formulas II and III wherein

$R^1$, $R^2$ are identical or different and independently hydrogen, fluorine, chlorine, methyl, methoxy, hydroxy, trifluoromethyl, nitro and

$R^3$, $R^4$ are identical or different and independently hydrogen, fluorine, chlorine, methoxy, hydroxy and

n is zero, while

A represents the L-glutathione radical.

3. Process according to claim 1, characterized in that it is started from compounds of formula II and III wherein

$R^1$, $R^2$ are identical or different and independently hydrogen, fluorine, chlorine, methyl, methoxy, hydroxy, trifluoromethyl, nitro and

$R^3$, $R^4$ are identical or different and independently hydrogen, fluorine, chlorine, methyl, methoxy, hydroxy and

n is zero or an integer from 1 to 4, while

A represents the L-cysteine radical.

**Revendications** (pour les Etats contractants : BE, CH, DE, FR, GB, IT, LI, LU, NL, SE)

1. Dérivés S-(carbamoyl-phénylsélényliques) du glutathion et d'acides α-aminomercaptocarboxyliques de formule générale I

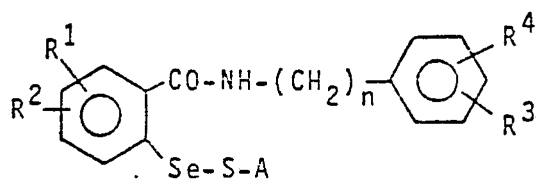

(I)

dans laquelle

$R^1$, $R^2$, $R^3$, $R^4$, ayant des significations identiques ou différentes, représentent chacun, indépendamment les uns des autres, l'hydrogène, un halogène, un groupe alkyle en C1-C4, alcoxy en C1-C4, trifluorométhyle, nitro, cyano, hydroxy, carboxy, (alcoxy en C1-C4)-carbonyle et

n est égal à 0 ou représente un nombre entier de 1 à 4, et

A représente le radical du glutathion ou un radical d'α-aminoacide, dans lequel le groupe carboxyle peut être estérifié par un alcool en C1-C3 et le groupe amino peut être acylé.

2. Dérivés S-(carbamoyl-phénylsélényliques) du L-glutathion de formule I, revendication 1, dans lesquels

$R^1$, $R^2$, ayant des significations identiques ou différentes, représentent chacun, indépendamment l'un de l'autre, l'hydrogène, le fluor, le chlore, un groupe méthyle, méthoxy, hydroxy, trifluorométhyle, nitro et

$R^3$, $R^4$, ayant des significations identiques ou différentes, représentent chacun, indépendamment l'un de l'autre, l'hydrogène, le fluor, le chlore, un groupe méthoxy, hydroxy et

n est égal à 0 et

A représente le radical du L-glutathion.

3. Dérivés S-(carbamoyl-phénylsélényliques) de la L-cystéine de formule I, revendication 1, dans laquelle

$R^1$, $R^2$, ayant des significations identiques ou différentes, représentent chacun, indépendamment l'un de l'autre, le fluor, le chlore, un groupe méthyle, méthoxy, hydroxy, trifluorométhyle, nitro et

$R^3$, $R^4$, ayant des significations identiques ou différentes, représentent chacun, indépendamment l'un de l'autre, l'hydrogène, le fluor, le chlore, un groupe méthyle, méthoxy, hydroxy et

n est égal à 0 ou représente un nombre entier de 1 à 4, et

A est le radical de la L-cystéine.

4. Procédé de préparation des composés de formule I selon les revendications 1 à 3, caractérisé en ce que l'on fait réagir sous agitation, à température ambiante, la 1,2-benzoisosélénazolone de formule II

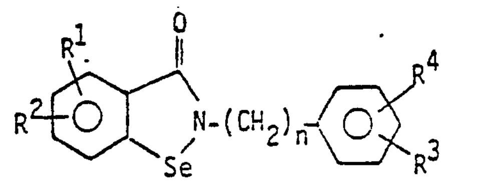

(II)

dans laquelle $R^1$, $R^2$, $R^3$, $R^4$ et n ont les significations indiquées dans la formule I, dissoute en quantité à peu près équimoléculaire dans un solvant miscible à l'eau, avec la solution aqueuse du composé particulier, contenant un groupe mercapto et répondant à la formule III

$$A{-}SH \qquad (III)$$

dans laquelle A a les significations indiquées en référence à la formule I.

5. Compositions pharmaceutiques caractérisées en ce qu'elles contiennent un composé de formule I selon les revendications 1 à 3, en tant que substance active, en mélange avec des produits auxiliaires et véhicules pharmaceutiques usuels.

**Revendications** (pour l'Etat contractant AT)

1. Procédé de préparation de dérivés S-(carbamoyl-phénylsélényliques) du glutathion et d'acides $\alpha$-amino-mercaptocarboxyliques répondant à la formule générale I

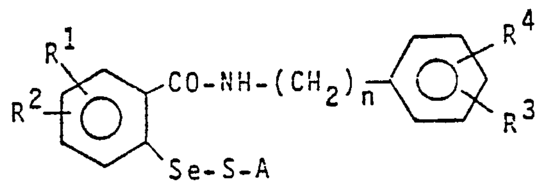

(I)

dans laquelle

$R^1$, $R^2$, $R^3$, $R^4$, ayant des significations identiques ou différentes, représentent chacun, indépendamment les uns des autres, l'hydrogène, un halogène, un groupe alkyle en C1-C4, alcoxy en C1-C4, trifluorométhyle, nitro, cyano, hydroxy, carboxy, (alcoxy en C1-C4)-carbonyle et

n est égal à 0 ou représente un nombre entier de 1 à 4, et

A représente le radical du glutathion ou un radical d'$\alpha$-aminoacide, dans lequel le groupe carboxyle peut être estérifié par un alcool en C1-C3 et le groupe amino peut être acylé,

caractérisé en ce que l'on fait réagir sous agitation à température ambiante la 1,2-benzoisosélénazolone de formule II

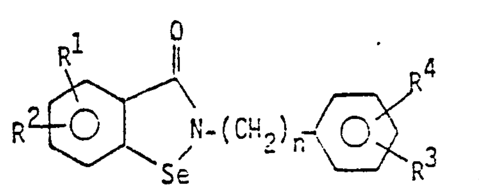

(II)

dans laquelle $R^1$, $R^2$, $R^3$, $R^4$ et n ont les significations indiquées en référence à la formule I, dissoute en quantité à peu près équimoléculaire dans un solvant miscible à l'eau, avec la solution aqueuse du composé particulier contenant un groupe mercapto et répondant à la formule III

$$A—SH \qquad (III)$$

dans laquelle A a les significations indiquées en référence à la formule I.

2. Procédé selon la revendication 1, caractérisé en ce que l'on part de composés de formules II et III dans lesquels

$R^1$, $R^2$, ayant des significations identiques ou différentes, représentent chacun, indépendamment l'un de l'autre, l'hydrogène, le fluor, le chlore, un groupe méthyle, méthoxy, hydroxy, trifluorométhyle, nitro et

$R^3$, $R^4$, ayant des significations identiques ou différentes, représentent chacun, indépendamment l'un de l'autre, l'hydrogène, le fluor, le chlore, un groupe méthoxy, hydroxy et

n est égal à 0, et

A représente le radical du L-glutathion.

3. Procédé selon la revendication 1, caractérisé en ce que l'on part de composés de formules II et III dans lesquels

$R^1$, $R^2$, ayant des significations identiques ou différentes, représentent chacun, indépendamment l'un de l'autre, l'hydrogène, le fluor, le chlore, un groupe méthyle, méthoxy, hydroxy, trifluorométhyle, nitro et

$R^3$, $R^4$, ayant des significations identiques ou différentes, représentent chacun, indépendamment l'un de l'autre, l'hydrogène, le fluor, le chlore, un groupe méthyle, méthoxy, hydroxy et

n est égal à 0 ou représente un nombre entier de 1 à 4 et

A représente le radical de la L-cystéine.